# EUROPÄISCHE PATENTANMELDUNG

(11) **EP 1 849 442 A1**
(43) Veröffentlichungstag der Anmeldung: **31.10.2007**
(21) Anmeldenummer: 07006423.3
(22) Anmeldetag: 28.03.2007
(51) Int. Cl.: A61F 5/11

(54) **Vorrichtung zur Nagelkorrektur mit hakenförmigen Krafteinleitungselementen**

(30) Priorität: 25.04.2006 DE 102006018987
(71) Anmelder: 3TO GmBH, 82041 Deisenhofen (DE)
(72) Erfinder: Sutor, Norbert, 82041 Deisenhofen (DE); Sutor, Johannes, 80339 München (DE)
(74) Vertreter: Müller, Bernhard

(57) **Zusammenfassung**

Es wird eine Vorrichtung zur Nagelkorrektur beschrieben, die mindestens zwei hakenförmigen Krafteinleitungselemente (1), die unter den Rand eines Zehennagels einhängbar sind, und mindestens ein Verbindungselement (2) aufweist. Dabei sind das oder die Verbindungselemente (2) flexibel ausgestaltet und fest an einem oder zwei Krafteinleitungselementen (1) angebracht , wobei das oder die Verbindungselemente so verkürzt werden können, dass auf die Krafteinleitungselemente (1) eine stufenlos einstellbare Zugkraft wirkt, die durch außermittigen Kraftangriff ein Biegemoment auf die Nagelplatte überträgt.

Wesentliche Vorteile der Vorrichtung bestehen unter anderem darin, dass auf das komplizierte Anbringen von losen Verbindungselementen verzichtet werden kann und das oder die Verbindungselemente aufgrund ihrer flexiblen Beschaffenheit sich beim Ausüben einer Zugkraft selbst an die Nageloberfläche anlegen.

## Beschreibung

Die Erfindung betrifft eine Vorrichtung zur Korrektur von menschlichen Näglen, insbesondere von Zehennägeln, gemäß Oberbegriff des Hauptanspruchs.

Zur Behandlung von eingewachsenen oder eingerollten Nägeln, insbesondere Großzehennägeln, wurden als Alternative zur schmerzhaften und mit langen Genesungszeiten verbundenen Operation (Emmert-Plastik) verschiedene Nagelkorrekturspangen entwickelt. Diese sogenannten Orthonyxiespangen reduzieren die Krümmung der Nagelplatte und entlasten dadurch den oftmals entzündeten Nagelfalz. Die Schmerzen werden deutlich gemindert und eine Abheilung ermöglicht.

Die Wirkung dieser Nagelkorrekturspangen beruht auf Rückstellkräften, außermittigen Zugkräften oder auf Kombinationen dieser beiden Mechanismen.

Folgende Vorrichtungen zur Nagelkorrektur sind bisher bekannt und werden zur Behandlung eingesetzt. Sie weisen jedoch entweder bezüglich ihrer Wirksamkeit oder ihrer Anwendung einige Nachteile auf:

### Drahtspange aus einem Teil (Fraserspange)

Die sogenannte Fraserspange ist eine einteilige Drahtspange, versehen mit Häkchen, welche unter den Nagelrand greifen und diesen mittels Rückstellkraft anheben. Diese Spange muss vom Therapeuten für jeden Patienten individuell angefertigt werden. Dies erfolgt unter erheblichem Zeitaufwand an einem zuvor anhand eines Abdruckes herzustellenden Modell. Die Vorrichtung wird von vorne auf den Nagel aufgeschoben, was eine exakte Anpassung auf die Nagelbreite erfordert und zudem ein erhebliches Verletzungsrisiko im engen oder gar entzündeten Nagelfalz darstellt. Die Wirkungskraft kann bei dieser Vorrichtung nur äußerst ungenau und nur durch sehr erfahrene Anwender eingestellt werden. Eine Einstellung der Kraft im angebrachten Zustand ist nicht möglich.

### Drahtspange aus 3 Teilen (2 Schenkel + 1 Schlaufe; DE 42 07 797 A1)

Eine Weiterentwicklung der Fraserspange stellt die dreiteilige Drahtspange dar, welche aus zwei mit Häkchen versehenen Schenkeln und einer in der Mitte anzubringenden Drahtschlaufe besteht. Der Therapeut versieht die teilweise vorgefertigten Spangenschenkel mit Häkchen (oder passt diese im Falle einer industriellen Vorfertigung derselben an die Nageldicke an) und formt die Spange entsprechend der Nagelkrümmung. Die Schenkel werden einzeln unter den Nagelrand eingehängt und mit der vorgefertigten Schlaufe verbunden, wodurch die Spangenbreite an die Nagelbreite angepasst und die Spangenkraft stufenlos in Absprache mit dem Patienten eingestellt werden kann.

Die Anwendung dieser Spangen erfordert großes Geschick des Therapeuten und macht eine spezielle Schulung unumgänglich. Zudem muss die Spange stets für die jeweilige Nagelbreite ausgesucht oder angepasst werden.

Auf Grund der geringen Abmessungen ist zudem das Anbringen der Mittelschlaufe relativ aufwändig.

### Klebespangen aus Kunststoff oder Metall (DE 37 08 811 A1; BS-Spange)

Auf die Rückstellkraft von Metall- oder Kunststoffelementen basierende Klebespangen werden im vorgespannten Zustand auf die Nagelplatte aufgeklebt. Dafür muss die Spange an den Nagel angedrückt werden, was bei ohnehin schmerzhaft eingewachsenen Nägeln mit erheblichen Schmerzen für den Patienten verbunden sein kann. Bei zu geringem Anpressdruck hingegen ist die Spange nicht ausreichend fixiert und kann sich wieder lösen.

Je nach System wird die Intensität der Spangenkraft durch die Vorauswahl verschiedener Querschnitte oder durch Verringerung der Spangendicke im aufgeklebten Zustand variiert. Dies kann jedoch weder definiert noch stufenlos geschehen und ist außerdem nicht reversibel. Der Kraftangriff erfolgt zudem nicht in den äußersten Randbereichen, da diese meist nicht sichtbar im Nagelfalz verborgen liegen.

### Nagelkorrekturspange bestehend aus 2 Ankerplättchen mit einem losen Zugelement

Eine Vorrichtung bestehend aus zwei, auf den Nagel aufgeklebten Ankern, welche durch ein loses Zugelement miteinander verbunden werden ist aus der DE 32 33 419 A1 bekannt. Hier können die Elemente zur Krafteinleitung im ungespannten Zustand auf die Nagelplatte geklebt werden. Die Einstellung der Spangenkraft erfolgt über ein elastisches Zugelement, wie zum Beispiel einen Gummiring, wobei die Intensität der Spangenkraft vom Abstand der Befestigungselemente und der Größe und Stärke des Zugelements abhängt und daher nicht genau und nicht stufenlos einstellbar ist.

Auf Grund der relativ hohen Ausführung der Ankerplatten kann durch enge Schuhe Druck auf den Nagel übertragen werden, zudem kann das nur lose befestigte Zugelement verloren werden.

Aufgabe der vorliegenden Erfindung ist es, eine Vorrichtung zur Nagelkorrektur zu schaffen, welche ohne aufwändige Anpassungsarbeiten mit nur einer vorgefertigten Größe universell und unkompliziert anwendbar ist, und mit der die erforderlichen Spangenkräfte in einfacher Weise stufenlos eingestellt werden können.

Im Laufe von Entwicklungsarbeiten im Rahmen der vorliegenden Erfindung wurde festgestellt, dass es von besonderem Vorteil ist, eine Nagelkorrekturvorrichtung so zu gestalten, dass durch Einwirkung einer stufenlos verstellbaren, definierbaren Kraft ein Biegemoment auf die Nagelplatte übertragen wird. Dabei hat es sich als besonders vorteilhaft erwiesen, wenn hakenförmige Krafteinleitungselemente mit mindestens einem flexibel ausgestalteten Verbindungselement fest verbunden sind. Eine stufenlos verstellbare Zugkraft kann durch Verkürzen des oder der Verbindungselemente ausgeübt werden.

Der Gegenstand der Erfindung ist im Anspruch 1 definiert.

Fortentwicklungen und besondere Ausführungsformen der Erfindung ergeben sich aus den Unteransprüchen.

Nachstehend wird die Erfindung beispielhaft anhand einiger Zeichnungen näher erläutert:

Dabei zeigen:
Figg. 1 und 2 eine erfindungsgemäße Vorrichtung zur Nagelkorrektur im montierten Zustand;
Figg. 3 und 4 Ausführungsbeispiele der erfindungsgemäßen Vorrichtung;
Figg. 5 bis 7 das Anbringen der Vorrichtung in der in Figur 3 dargestellten Ausführung;
Figg. 8 bis 11 Ausführungsbeispiele der Krafteinleitungselemente;

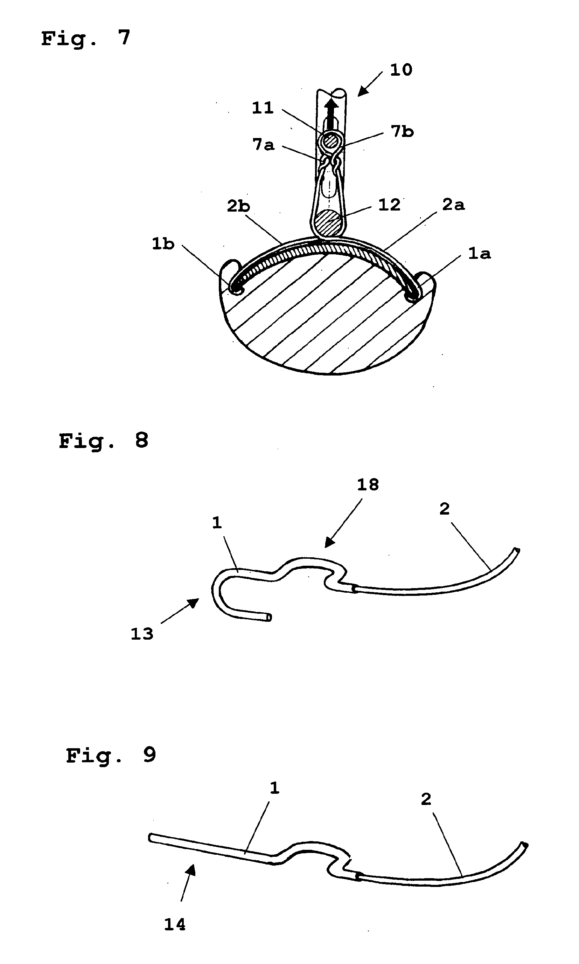

Figg. 8 bis 11 alternative Möglichkeiten zur Verbindung des Krafteinleitungselements mit dem Verbindungselement;
Figg. 8, 10 und 11 Maßnahmen zur Kippstabilisierung der erfindungsgemäßen Vorrichtung;

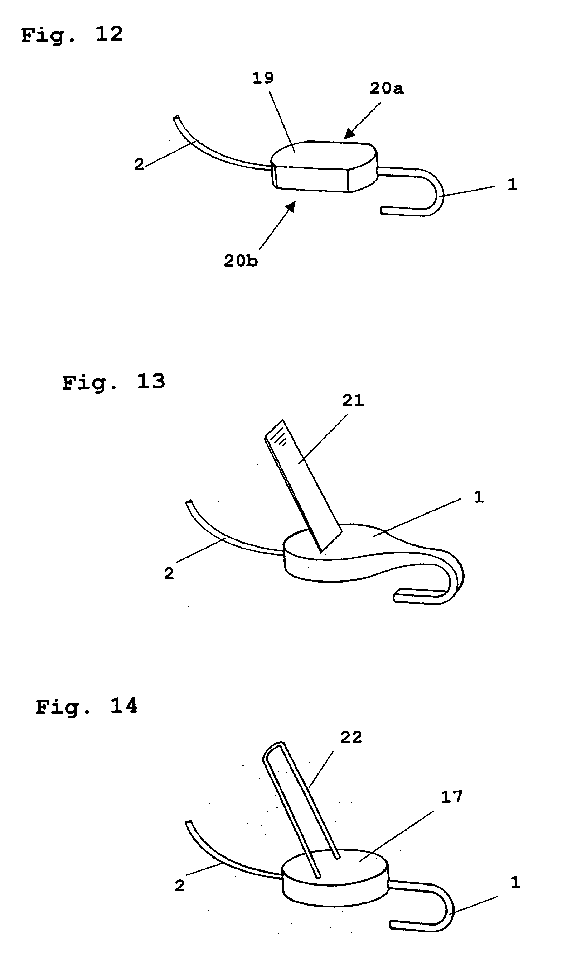

Figg. 12 bis 14 Handhabungshilfen für die Anwendung der Vorrichtung zur Nagekorrektur;

Nachstehend wird auf die in den Figuren dargestellten Ausführungsformen näher eingegangen.

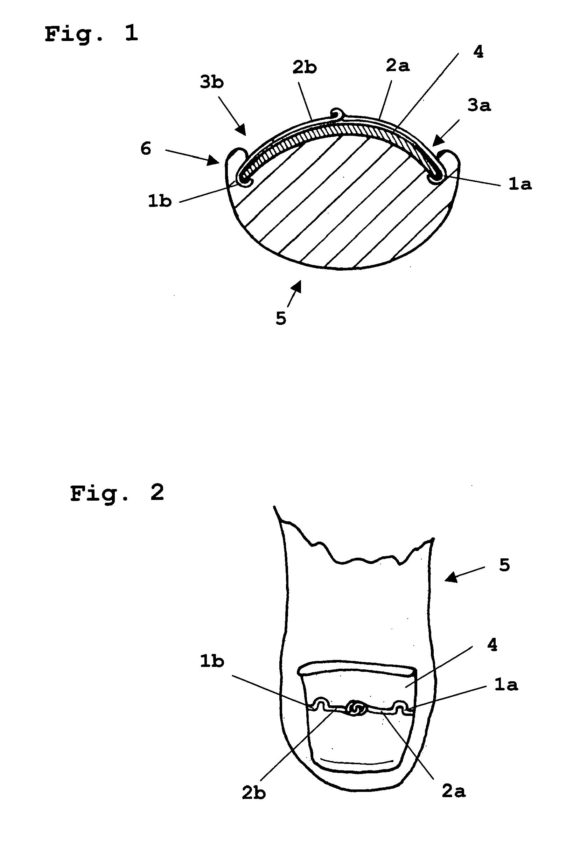

Figur 1 und 2 zeigen die erfindungsgemäße Vorrichtung im auf den Nagel montierten Zustand

Zur Behandlung des eingewachsenen Nagels (4) einer Zehe (5) sollen die Nagelränder (3a, 3b) minimal angehoben werden, um den Nagelfalz (6) zu entlasten. Hierfür werden zwei hakenförmige Krafteinleitungselemente (1a, 1b) unter die Nagelränder (3a, 3b) eingehängt und über die Verbindungselemente (2a, 2b) aus Draht miteinander verbunden.

Durch das definierte und stufenlose Verkürzen der freien Länge der Verbindungselemente (2a, 2b) und die Fixierung der eingestellten Position wird eine Zugkraft zwischen den Krafteinleitungselementen (1a, 1b) erzeugt, welche außermittig am Nagel (4) angreift. Auf den Nagel (4) wirkt dadurch ein Biegemoment, die Nagelränder (3a, 3b) werden angehoben.

### Unterschiedliche Ausführungen der Vorrichtung zur Nagelkorrektur

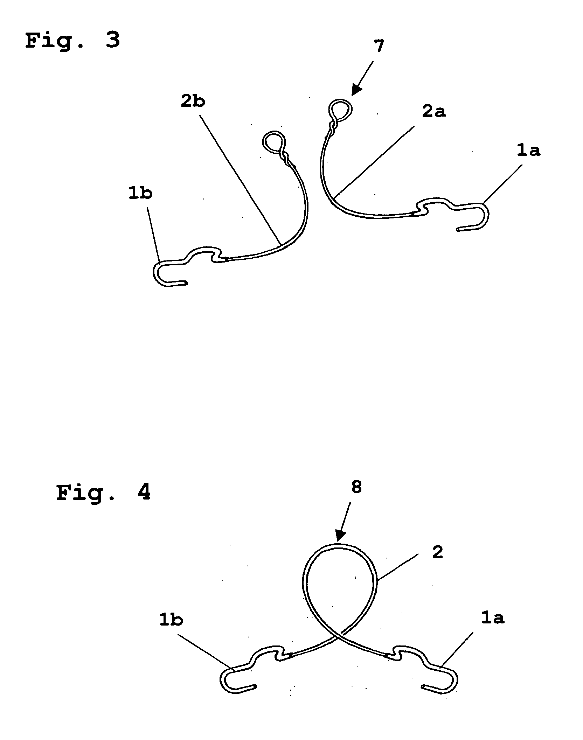

Die Figuren 3 und 4 zeigen unterschiedliche Ausführungen der erfindungsgemäßen Vorrichtung

In Figur 3 ist eine zweigeteilte Ausführung der erfindungsgemäßen Vorrichtung zur Nagelkorrektur dargestellt. Mit jedem hakenförmigen Krafteinleitungselement (1a, 1b) ist je ein Verbindungselement (2a, 2b) fest verbunden. Für die Nagelkorrektur kommen mindestens zwei dieser Kombinationen zum Einsatz, wobei unter jeden der beiden Nagelränder (3a, 3b) mindestens ein Haken eingehängt wird.

Die Verbindungselemente (2a, 2b) können an ihren Enden mit Haken oder Ösen (7) versehen sein, um die Handhabung mit Werkzeugen zu erleichtern.

Alternativ kann die Vorrichtung zur Nagelkorrektur, wie in Figur 4 dargestellt, einteilig ausgeführt sein. Hierbei sind zwei hakenförmige Krafteinleitungselemente (1a, 1b) mit je einem Ende eines gemeinsamen Verbindungselementes (2) fest verbunden. Das Verbindungselement (2) kann zur vereinfachten Handhabung in Form einer Schlinge (8) ausgeführt sein.

### Anbringen der Vorrichtung zur Nagelkorrektur

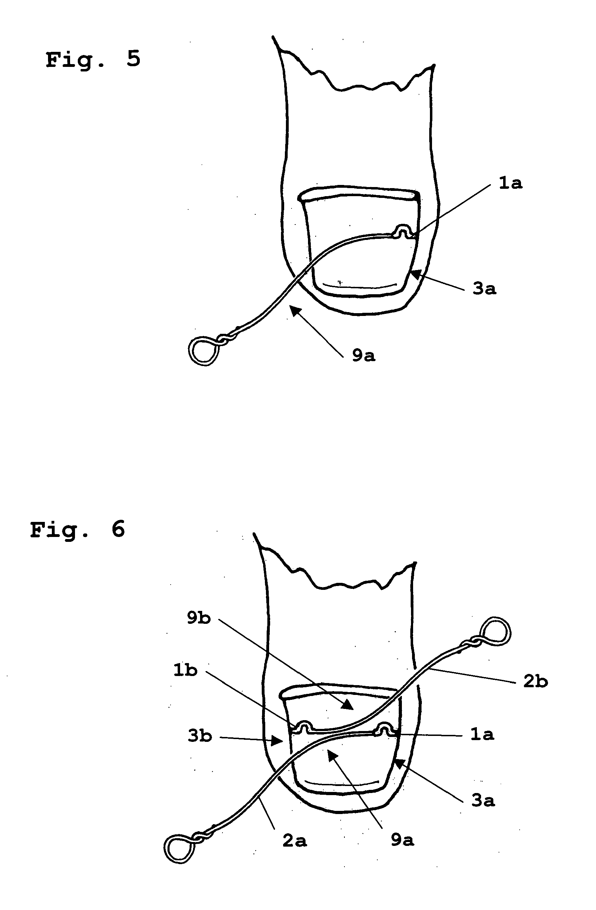

In den Figuren 5 bis 7 ist das Anbringen der erfindungsgemäßen Vorrichtung in der in Figur 3 dargestellten Ausführung an den Nagel dargestellt.

Zunächst wird, wie in Figur 5 dargestellt, die erste Spangenhälfte (9a) mit dem Häkchen (1a) unter den rechten Nagelrand (3a) eingehängt. Anschließend wird mit der zweiten Spangenhälfte (9b) am linken Nagelrand (3b) ebenso verfahren. Hierbei kommt, wie in Figur 6 ersichtlich, das Verbindungselement (2b) der zweiten Spangenhälfte (9b) aus Sicht des Anwenders hinter dem Verbindungselement (2a) der ersten Spangenhälfte (9a) zu liegen.

Figur 7 zeigt das Spannen der Vorrichtung mittels eines entsprechenden Werkzeuges (10). Die Ösen (7a, 7b) werden hierfür in den oberen Zapfen (11) eingehängt, die Verbindungselemente (2a, 2b) um den unteren Zapfen (12) herumgeführt. Durch stufenlose Vergrößerung des Abstandes der beiden Zapfen (11, 12) wird der Abstand zwischen den beiden hakenförmigen Elementen kontinuierlich verkürzt, bis die gewünschte Zugkraft erreicht ist.

Zur Lagefixierung der eingestellten Länge wird das Werkzeug (10) um mindestens 180° im Uhrzeigersinn gedreht wodurch die beiden Verbindungselemente (2a, 2b) dauerhaft miteinander verbunden werden.

Außer dieser formschlüssigen Verbindung der Verbindungsteile ist ebenso eine kraft- oder stoffschlüssige Verbindung möglich. Hierfür können Verbindungselemente der unterschiedlichsten Materialien, wie beispielsweise Draht, Kunststoff, Fäden o.ä. verwendet werden.

Figur 1 zeigt die fertig angebrachte Vorrichtung zur Nagelkorrektur nach dem Abtrennen der überschüssigen Drahtreste der Verbindungselemente.

Das Aufbringen der Spannung und die Fixierung der Lage können ebenso durch das Verdrillen der Verbindungselemente erfolgen.

Zur endgültigen Fixierung und zum Schutz vor Verletzungen wird die Verbindungsstelle mit aushärtendem Kunststoffmaterial abgedeckt.

### Ausführungsbeispiele der Krafteinleitungselemente

Die Figuren 8 bis 11 zeigen beispielhaft einige Ausführungen der Krafteinleitungselemente (1).

In der in Figur 8 dargestellten Ausführung besteht das Krafteinleitungselement (1) aus federhartem Draht, welcher in Form eines Hakens (13) vorliegt. Der in Figur 8 abgebildete Haken (13) ist grob vorgebogen und wird vom Anwender mittels einer Spezialzange (bekannt aus GBM 20 2005 014 365.0) entsprechend der Nageldicke nachgeformt. Ebenso ist eine Ausführung mit bereits fertig gebogenem Haken denkbar.

Figur 9 zeigt die Vorrichtung in einer Ausführung, bei welcher das Krafteinleitungselement (1) noch nicht mit einem Haken versehen ist, sondern noch ein gerades Stück (14) federharten Drahtes darstellt. Der Haken wird hier individuell vom Anwender unter Zuhilfenahme von Werkzeugen angefertigt.

Durch eine Variation der Länge des federharten Bereichs des Krafteinleitungselementes (1) kann die Wirkung der Nagelkorrekturspange wahlweise durch reine außermittige Zugkraft oder eine Kombination von Zugkraft und Hebelwirkung realisiert werden.

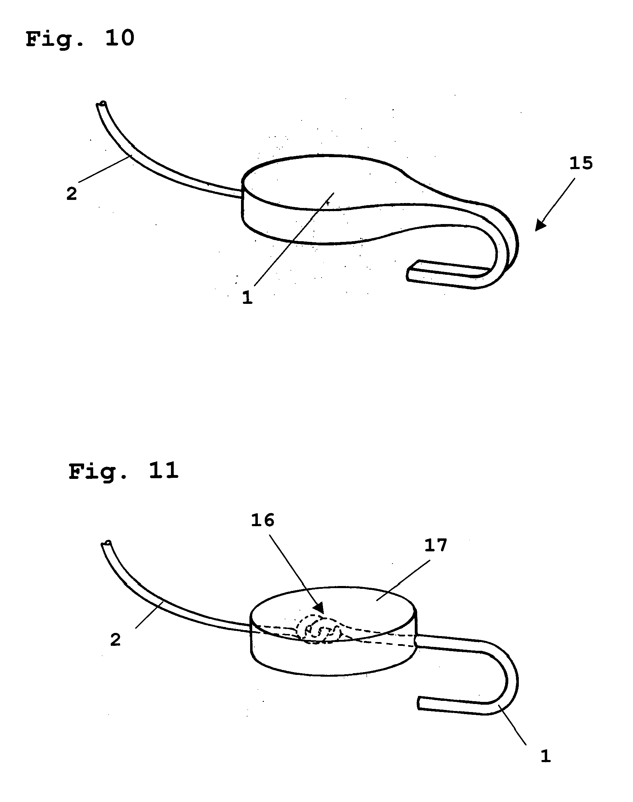

In Figur 10 ist ein Krafteinleitungselement (1) als Formteil dargestellt, welches an seinem Ende (15) hakenförmig ausgebildet ist. Dieses Formteil kann aus Materialien wie Kunststoff, Metall oder Keramik hergestellt sein und verschiedenste Formen aufweisen. Das Krafteinleitungselement kann außerdem eine an die Anatomie des Nagels angepasste Auflagefläche aufweisen, welche zur Verbesserung der Handhabbarkeit mit dem Nagel verklebt werden könnte.

Die feste Verbindung mit dem Verbindungselement (2) kann durch Stoff-, Form- oder Kraftschluss hergestellt werden.

Zur besseren Handhabbarkeit kann das Krafteinleitungselement mit einem Ansatz zur Manipulation mit Werkzeugen oder aber zur werkzeuglosen Manipulation versehen sein.

### Alternative Möglichkeiten zur Verbindung der Krafteinleitungselemente mit den Verbindungselementen

Die Figuren 8, 10 und 11 zeigen beispielhaft einige Möglichkeiten zur festen Verbindung zwischen Krafteinleitungselementen und Verbindungselementen.

Figur 8 zeigt den Endbereich der erfindungsgemäßen Vorrichtung zur Nagelkorrektur gemäß der in Figur 3 dargestellten Ausführung. Die Vorrichtung besteht in dieser Ausführung vollständig aus Draht, wobei dieser im Bereich des Krafteinleitungselementes (1) federhart und damit elastisch verformbar, im Bereich des Verbindungselementes (2) hingegen schlussgeglüht, d.h. plastisch verformbar ausgeführt ist.

Vorteilhaft erweist sich zudem eine unterschiedliche Drahtstärke, welche für das Krafteinleitungselement (1) im Bereich von ca. 0,4 - 0,5 mm Durchmesser und für das Verbindungselement (2) bei ca. 0,3 - 0,4 mm Durchmesser liegt.

Krafteinleitungselement (1) und Verbindungselement (2) können aus einem Stück Draht hergestellt sein, welches in unterschiedlichen Bereichen unterschiedlichen Wärmebehandlungen unterzogen wurde und dessen Durchmesser im Bereich des Verbindungsteiles (2) gegebenenfalls spanend oder spanlos reduziert wurde. Alternativ können zwei Drähte verschiedener Stärke und unterschiedlichen Gefügezustandes durch Schweißen oder Löten miteinander verbunden werden.

Figur 10 zeigt die erfindungsgemäße Vorrichtung mit einem als Formteil ausgeführten Krafteinleitungselement (1). Das Verbindungselement (2) aus Draht oder Kunststoff kann in dieses Formteil eingegossen, beziehungsweise das Formteil an das Verbindungselement (2) angespritzt werden. Das Verbindungselement kann zur Erhöhung der Verbindungsfestigkeit mit dem Krafteinleitungselement (1) an seinem Ende beispielsweise in Form eines Hakens ausgebildet sein.

Figur 11 zeigt eine Ausführung mit formschlüssiger Verbindung zwischen Krafteinleitungselement (1) und Verbindungselement (2), wobei beide Elemente aus Draht bestehen. Der federharte Draht des Krafteinleitungselements (1) und der weiche Draht des Verbindungselementes (2) können durch Verhaken, Umwickeln oder Verdrillen miteinander verbunden werden. Zum Schutz vor Lösen dieser Verbindung kann die Fügestelle (16) mit Kunststoff oder anderen Materialien eingegossen oder ummantelt werden. Diese Umhüllung (17) kann in beliebiger Form ausgebildet sein und gleichzeitig zur verbesserten Handhabbarkeit und Lagestabilisierung der Vorrichtung dienen.

### Maßnahmen zur Lagestabilisierung

Um ein Herausdrehen des Hakens unter dem Nagelrand während dem Anbringen oder im Laufe der Therapie zu vermeiden, kann die erfindungsgemäße Vorrichtung Elemente zur seitlichen Stabilisierung aufweisen.

Dies kann, wie in Figur 8 dargestellt mit Hilfe einer oder mehrerer beliebig geformter Ausbuchtungen (18) im Draht geschehen, wobei dieser in eine oder beide Richtungen ausgebuchtet sein kann.

Gleichzeitig wirkt diese Maßnahme als elastische Komponente in der Zugrichtung, was sich vorteilhaft auf die Therapie auswirken könnte.

In der in Figur 11 abgebildeten Ausführung wird die Ummantelung (17) der Fügestelle (16) gleichzeitig zur Lagestabilisierung genutzt, indem diese an ihrer, dem Nagel zugewandten Seite flach ausgeführt ist.

### Manipulationshilfen

Zur Vereinfachung der Handhabung beim Anbringen der Vorrichtung zur Nagelkorrektur kann diese mit Manipulationshilfen versehen sein. Diese können in das Krafteinleitungselement (1) integriert sein oder an der Fügestelle zwischen Krafteinleitungselement (1) und Verbindungselement (2) vorgesehen werden. Die Manipulationshilfen können entweder zur Handhabung mit Werkzeugen oder zur werkzeuglosen Handhabung dienen.

Sowohl die in Figur 10 dargestellte Ausführung des Krafteinleitungselementes als Formteil als auch die Ummantelung (17) der Fügestelle (16) der in Figur 11 dargestellten Ausführung bieten beispielsweise als Kunststoff-Spritzgussteil mehrere Möglichkeiten, Handhabungshilfen zu integrieren.

Beispielsweise kann ein derartiges Formteil (19) zum Greifen mit einer Pinzette, wie in Figur 12 dargestellt, an den Seitenflächen parallele Flanken (20a, 20b) aufweisen, welche zusätzlich konkav gewölbt sein können.

Zur vorübergehenden Fixierung der Krafteinleitungselemente während des Anbringens der Vorrichtung zur Nagelkorrektur kann die nagelseitige Oberfläche der Ummantelung der Fügestelle (17; Fig. 11) mit Haftklebstoff versehen werden.

Figur 13 und 14 zeigen Manipulationshilfen zum werkzeuglosen Einhängen der Krafteinleitungselemente.

In Figur 13 ist das als Formteil ausgeführte Krafteinleitungselement (1) mit einem Ansatz (21) versehen, welcher mit den Fingern gegriffen werden kann und nach dem Anbringen der Spange abgetrennt wird.

In Figur 14 dient ein Drahtbügel (22) als Greifhilfe. Hierfür kann der Draht des Krafteinleitungselementes (1) über die Fügestelle aus der Ummantelung (17) hinaus verlängert werden und als Greifhilfe dienen. Der Drahtbügel (22) wird nach dem Einhängen abgetrennt.

### Vorteile der erfindungsgemäßen Vorrichtung zur Nagelkorrektur

Gegenüber den bisher bekannten, herkömmlichen Verfahren und Vorrichtungen weist die erfindungsgemäße Vorrichtung zur Nagelkorrektur einige Vorteile auf.

Durch ihre schnelle und einfache Anwendung wird diese Nagelkorrekturspange der Anforderung der Wirtschaftlichkeit in der ärztlichen, podologischen und kosmetischen Praxis gerecht.

Durch die bereits bestehende feste Verbindung der Krafteinleitungselemente mit einem beziehungsweise mehreren Verbindungselementen kann hier auf das komplizierte Anbringen eines losen Verbindungselementes, wie es bei herkömmlichen, dreigeteilten Drahtspangen der Fall ist, verzichtet werden.

Durch ein im Verhältnis zum Krafteinleitungselement sehr langes Verbindungselement kann außerdem auf eine Anpassung der Nagelkorrekturspange an die Nagelkrümmung verzichtet werden, da die Vorrichtung nahezu im gesamten Auflagebereich auf der Nagelplatte aus weichem, flexiblem Material besteht, welches sich beim Aufbringen der Zugkraft selbst an die Nageloberfläche anlegt.

Hierdurch wird der Einsatz eines bereits teilweise oder vollständig geformten Häkchens möglich, wobei hierfür auf Grund des im Verhältnis sehr kurzen Krafteinleitungselementes nur eine Spangengröße erforderlich ist.

Durch die im Bereich des Verbindungselementes flexible Ausführung der Nagelkorrekturspange können die Häkchen auch bei einer einteiligen Ausführung der Vorrichtung direkt an der Wirkstelle unter den Nagelrand eingehängt werden und müssen nicht, wie die bekannte einteilige Drahtspange von vorne auf den Nagel aufgeschoben werden. Zusätzliche Schmerzen beim Anbringen der Vorrichtung werden hierdurch vermieden.

Bei der Aktivierung der Nagelkorrekturspange wird die Zugkraft direkt auf das Verbindungselement aufgebracht und nicht, wie bei bekannten Spangen durch ein zusätzliches Spannelement. Durch das kontinuierliche Verkürzen des Verbindungselements kann die Zugkraft und damit das Biegemoment auf den Nagel stufenlos eingestellt werden. Dies kann in Absprache mit dem Patienten erfolgen und ist bis zum Kürzen des Verbindungselementes (siehe Fig. 1) reversibel.

Auf Grund ihrer sehr flachen Bauweise wird diese Vorrichtung zur Nagelkorrektur auch in engen Schuhen nicht als störend empfunden.

## Patentansprüche

1. Vorrichtung zur Korrektur von menschlichen Nägeln, insbesondere Zehennägeln, die mindestens zwei hakenförmigen Krafteinleitungselemente (1), die unter den Rand eines Nagels einhängbar sind, und mindestens ein Verbindungselement (2) aufweist, **dadurch gekennzeichnet, dass** das oder die Verbindungselemente (2) flexibel sind und fest an einem oder zwei Krafteinleitungselementen (1) angebracht sind, wobei das oder die Verbindungselemente so verkürzt werden können, dass auf die Krafteinleitungselemente (1) eine stufenlos einstellbare Zugkraft wirkt, die durch außermittigen Kraftangriff ein Biegemoment auf die Nagelplatte überträgt.

2. Vorrichtung nach Anspruch 1, **dadurch gekennzeichnet, dass** die Vorrichtung mehrteilig ist.

3. Vorrichtung nach Anspruch 1, **dadurch gekennzeichnet, dass** die Vorrichtung einteilig ist

4. Vorrichtung nach einem der vorstehenden Ansprüche, **dadurch gekennzeichnet, dass** die Haken der Krafteinleitungselemente bei Bedarf formbar sind.

5. Vorrichtung nach einem der Ansprüche 1 bis 3, **dadurch gekennzeichnet, dass** die Haken der Krafteinleitungselemente bereits ganz oder teilweise vorgeformt sind.

6. Vorrichtung nach einem der vorstehenden Ansprüche, **dadurch gekennzeichnet, dass** das Krafteinleitungselement und/oder das Verbindungselement elastische Komponenten aufweisen.

7. Vorrichtung nach einem der vorstehenden Ansprüche, **dadurch gekennzeichnet, dass** die Zugkraft durch Verdrillen des Verbindungselementes ausübbar ist.

8. Vorrichtung nach einem der Ansprüche 1 bis 6, **dadurch gekennzeichnet, dass** die Zugkraft mit Hilfe eines entsprechenden Werkzeuges ausgeübt wird.

9. Vorrichtung nach einem der vorstehenden Ansprüche, **dadurch gekennzeichnet, dass** die nach Ausübung der Zugkraft erreichte Position durch Formschluss fixierbar ist.

10. Vorrichtung nach einem der vorstehenden Ansprüche, **dadurch gekennzeichnet, dass** die Vorrichtung Elemente zur Lagestabilisierung aufweist.

11. Vorrichtung nach einem der vorstehenden Ansprüche, **dadurch gekennzeichnet, dass** an der Vorrichtung Elemente zur Manipulationshilfe vorgesehen sind.

12. Vorrichtung nach einem der vorstehenden Ansprüche, **dadurch gekennzeichnet, dass** im Bereich der Krafteinleitungselemente zur vorübergehenden Fixierung mit Haftklebstoff versehene Auflageflächen vorgesehen sind.

13. Vorrichtung nach einem der vorstehenden Ansprüche, **dadurch gekennzeichnet, dass** die Krafteinleitungselemente aus federhartem Draht und dass das oder die Verbindungselemente aus schlussgeglühtem, weichem Draht bestehen.
